Europäisches Patentamt

European Patent Office

Office européen des brevets

(11) Veröffentlichungsnummer: **0 210 557 B1**

(12) **EUROPÄISCHE PATENTSCHRIFT**

(45) Veröffentlichungstag der Patentschrift:
**15.02.89**

(21) Anmeldenummer: **86109896.0**

(22) Anmeldetag: **18.07.86**

(51) Int. Cl.⁴: **A61M 5/14**, H02K 49/00

(54) **Magnetkupplung mit integrierter magnetischer Lagerentlastung.**

(30) Priorität: **01.08.85 DE 3527687**

(43) Veröffentlichungstag der Anmeldung:
**04.02.87 Patentblatt 87/6**

(45) Bekanntmachung des Hinweises auf die Patenterteilung:
**15.02.89 Patentblatt 89/7**

(84) Benannte Vertragsstaaten:
**DE FR GB IT**

(56) Entgegenhaltungen:
**EP-A- 0 034 992**
**DE-A- 3 338 002**
**DE-B- 1 165 144**
**US-A- 2 436 939**

(73) Patentinhaber: **Siemens Aktiengesellschaft Berlin und München, Wittelsbacherplatz 2, D-8000 München 2(DE)**

(72) Erfinder: **Obermann, Peter, Am Färberhof 12, D-8520 Erlangen(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Stirnmagnetkupplung versehen mit mindestens einem Magneten zur Übertragung von Drehmomenten oder Kräften von einem ersten in einem zweiten Raum, wobei die Räume voneinander durch eine Trennwand getrennt sind.

Eine Trennung wird benötigt, wenn man von einem ersten Raum mit bestimmten raumklimatischen Bedingungen Energie oder Information auf mechanische Weise, also an Drehmomente oder Kräfte und Drehwinkel oder Wege gekoppelt, in einen zweiten Raum überträgt. In dem zweiten Raum herrschen dabei andere klimatische Bedingungen, die von den im ersten Raum vorliegenden raumklimatischen Bedingungen verschieden und mit diesen unvereinbar sind. Eine derartige Unvereinbarkeit verscheidener raumklimatischer Bedingungen in verschiedenen Räumen tritt vornehmlich beim Umgang mit gasförmigen oder flüssigen Medien auf. Insbesonders, falls diese zusätzlich noch korrosiv, aggressiv, giftig, explosiv, radioaktiv sein sollten oder extremen Reinheitsanforderungen unterliegen sollten.

Ein Anwendungsfall zur Korrosion liegt z.B. bei einem Medikamentendosiergerät vor. Dort werden hohe Anforderungen bezüglich der Zuverlässigkeit und der Lebensdauer gestellt. Dieses gilt noch verstärkt für implantierbare Medikamentendosiergeräte. Aufgrund der Feuchtigkeit, die von einem flüssigen oder auch gelartigen Medikament ausgeht, werden empfindliche Bauteile, wie z.B. der Motor, elektrische Kontakte oder die Batterie in ihrer Leistung oder Funktionsweise beeinträchtigt.

Um hier Abhilfe zu schaffen, ist es zweckmäßig, das Medikamentendosiergerät in zwei Räume zu unterteilen. Die Unterteilung erfolgt zum einen in einen Trockenraum, in welchem die vor Feuchtigkeit zu schützenden Teile des Dosiergeräts untergebrachts sind, z.B. der elektrische Motor, und zum anderen in einem Feuchtraum, in welchem die mit der Flüssigkeit in Kontakt kommenden Teile, wie z.B. ein Pumpschlauch, ein Medikamentenvorratsbehälter, ein Pumpgetriebe etc. untergebracht sind. Die beiden Räume sind durch eine Trennwand voneinander getrennt und hermetisch nach außen abgeschlossen. Der Motor und das Pumpgetriebe liegen also nicht im selben Raum, wodurch eine Kraftübertragung zwischen den beiden Räumen nötig ist. Diese wird von einer indirekten Magnetdrehkupplung, die über magnetische Feldwirkung ein Drehmoment von dem ersten in den zweiten Raum überträgt, bereitgestellt. Eine solche Dosiervorrichtung ist aus der DE-OS 3 338 002 bekannt.

Es hat sich gezeigt, daß aufgrund der Anziehung über Axialkräfte der beiden Teile der Magnetdrehkupplung eine aufwendige Lagerkonstruktion nötig ist, um einen definierten Abstand der sich drehenden Magnete von der Trennwand sicherzustellen. Außerdem ist oft durch die stärker Reibung der Kraftaufwand für den Antrieb erheblich größer, als wenn die Axialkräfte kompensiert wären.

Um das Auftreten von Axialkräften bei Magnetkupplungen mit Permanentmagneten zur Übertragung von Drehkräften von einem antreibenden Teil auf ein angetriebenes Teil zu vermeiden, ist es aus der EP-A 0 034 992 bereits bekannt, auf der Achse des treibenden bzw. des angetriebenen Teiles zwei ringförmige Permanentmagnete axial versetzt anzuordnen und die Achse des jeweils anderen Teiles mit einem hohlzylinderförmigen Endstück zu versehen, dessen Innendurchmesser größer ist als der Außendurchmesser der beiden genannten Magnete und an dessen Ende einen weiteren Ringmagneten derart anzuordnen, daß dieser in axialer Richtung symmetrisch zwischen den beiden anderen Magneten liegt.

Wegen der Überdeckung der beiden Teile in axialer Richtung eignet sich diese Anordnung nicht für eine Übertragung von Drehkräften durch die Trennwand zweier in sich abgeschlossener Räume.

Aufgabe der Erfindung ist es daher, eine Stirnmagnetkupplung der eingangs genannten Art mit mindestens einem Magneten so auszubilden, daß die magnetische Axialkraft zufriedenstellend kompensiert und dennoch dafür wenig Platz beansprucht wird.

Diese Aufgabe wird durch die im Patentanspruch 1 angegebene Erfindung gelöst.

Dadurch ist erreicht, daß keine zusätzlichen Baukomponenten eingebaut werden müssen, durch welche die Kompensationskraft zur Lagerentlastung aufgebracht werden muß. Das ohnehin bei dem Magneten der Magnetkupplung vorhandene Magnetfeld wird also nicht nur zur Drehmoment- oder Kraftübertragung herangezogen, sondern gleichzeitig zur Kompensation der Axialkraft, also zur Lagerentlastung benutzt. Durch diese Maßnahme ergibt sich ein leichter Lauf der Magnetkupplung, da im wesentlichen nur noch die Tangentialkraft zur reinen Drehmoment- oder Kraftübertragung aufgebracht werden muß. Die Magnetkupplung mit integrierter magnetischer Lagerentlastung führt zu einer im Vergleich zu konventionellen Magnetkupplungen relativ kleinen und konstruktiv einfachen Bauform, die der fortschreitenden Forderung nach Miniaturisierung entgegenkommt.

In einer vorteilhaften Weiterbildung der Erfindung ist die Platte stationär an einem Gehäuseteil oder einem am Gehäuse befestigten Geräteteil angebracht.

In einer weiteren Ausführungsform ist das Geräteteil ein mit der Magnetkupplung verbundener Antrieb und eine Stirnseite des Antriebsgehäuses bildet die Platte. Durch diese Maßnahme ist eine Lageentlastung erreicht, ohne daß zusätzlicher Platz beansprucht wird.

Weitere Ausgestaltungen der Erfindung ergeben sich aus dem im folgenden beschriebenen Ausführungsbeispiel in Verbindung mit den Unteransprüchen. Es zeigen:

Fig. 1 einen Schnitt durch eine schematische Darstellung einer Stirnmagnetdrehkupplung mit jeweils zwei Magneten in jedem Raum und

Fig. 2 einen Schnitt II–II durch die Stirndrehkupplung gemäß Figur 1.

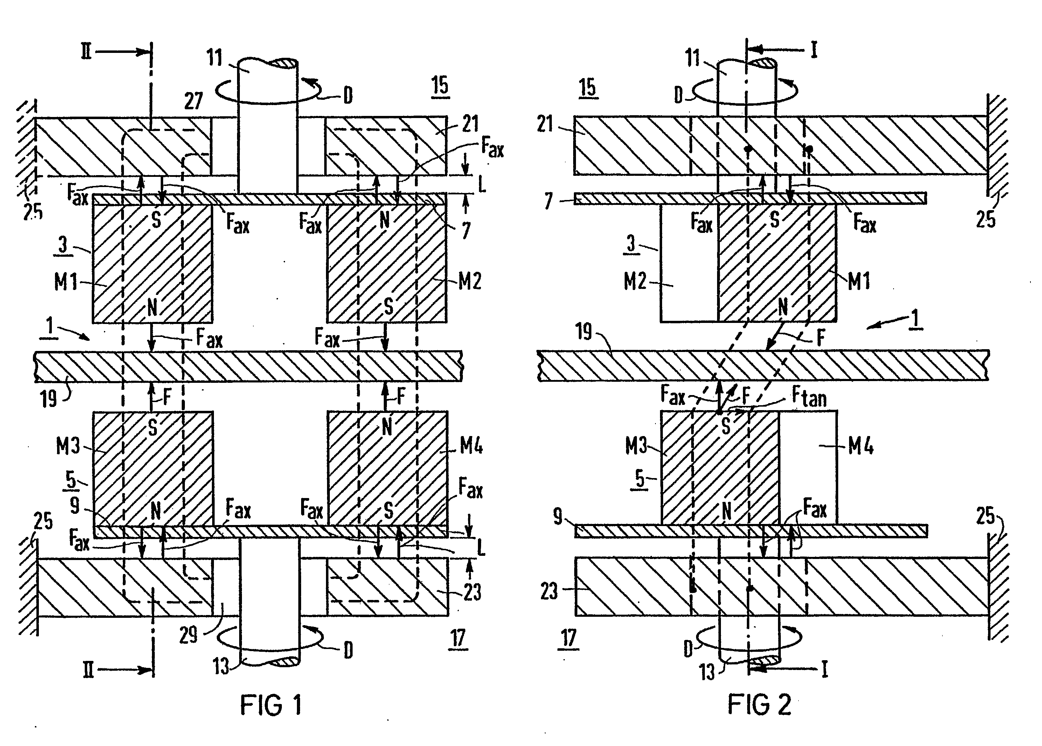

In den Figuren 1 und 2 ist der Übersichtlichkeit halber eine Stirnmagnetdrehkupplung 1 lediglich mit ihren wesentlichen Elementen dargestellt. Die Stirnmagnetdrehkupplung 1 umfaßt einen Antriebsteil 3 und einen Abtriebs- oder Getriebeteil 5. Der Antriebs- und der Getriebeteil 3 bzw. 5 sind im wesentlichen identisch ausgebildet und befinden sich beispielsweise in dem Innenraum eines implantierbaren Dosiergerätes. Sie umfassen jeweils zwei Magnete M1, M2 bzw. M3, M4, jeweils eine Magnetträgerplatte 7 bzw. 9 und jeweils eine Welle 11 bzw. 13, welche über die Magnetträgerplatten 7, 9 fest mit den Magneten M1 bis M4 verbunden sind. Die Welle 11 ist an einen (nicht gezeigten) Antrieb angeschlossen, welcher das Antriebsteil 3 entlang des Einfachpfeils D in Rotation versetzt.

Die Welle 13 ist an einen Abtrieb oder ein Getriebe (nicht gezeigt) angeschlossen, welches den eigentlichen Dosiervorgang bewirkt. So kann z.B. mit der Welle 13 ein Rollenträger einer an sich bekannten Peristaltikpumpe verbunden sein. Bei Drehung der Welle 13 wird der Rollenträger bewegt und fördert durch Abquetschen des eingelegten Schlauches das in dem Schlauch befindliche Medikament zu einer Austrittsöffnung.

Der Antriebsteil 3 befindet sich in einem ersten geschlossenen Raum 15, welcher als Trockenraum bezeichnet werden kann. In diesem ersten Raum 15 sind Bauteile und Vorrichtungen untergebracht, die vor den Eigenschaften der in Raum 17 befindlichen Medien zu schützen sind. Solche Bauteile und Vorrichtungen können z.B. Motoren, Batterien, elektrische oder mechanische Bauelemente oder Meßwerke sein. Der Abtriebsteil 5 befindet sich in einem zweiten Raum 17, welcher als Medienraum zu bezeichnen ist. Dort sind im wesentlichen diejenigen Bauteile untergebracht, die mit dem eingesetzten Medium in Kontakt stehen. Solche Bauteile bilden z.B. das eigentliche Prozeßgetriebe. Der erste Raum 15 ist von dem zweiten Raum 17 durch eine Trennwand 19 hermetisch getrennt.

Dicht oberhalb der Magnetträgerplatten 7, 9 befindet sich als magnetischer Rückschluß jeweils eine Platte 21 bzw. 23, die aus magnetisierbarem Material besteht, z.B. aus Nickel oder Weicheisen, und fest mit dem Gehäuse der Dosiervorrichtung oder einem anderen stationären Teil der Dosiervorrichtung verbunden ist. Dieses ist durch die Auflager 25 angedeutet. Die Platten 21, 23 sind an der Stelle, an welcher die Wellen 11 bzw. 13 durchgeführt werden müssen, mit einer Ausnehmung oder Aussparung 27 bzw. 29 versehen. Die Platten 21, 23 weisen einen geringen Luftspalt zur Magnetträgerplatte 7 bzw. 9 auf. Die Magnetträgerplatte 7, 9 dient lediglich dem mechanischen Schutz der Magnete M1 bis M4, die in Gießharz eingebettet oder anderweitig gekapselt sein können. Sie kann von der Funktionsweise und dem magnetischen Fluß der Vorrichtung her gesehen auch wegfallen. Dann ist ein Luftspalt L direkt zwischen den Platten 21, 23 und den Magneten M1, M2 bzw. M3, M4 einzustellen. Die Länge dieses Luftspaltes L sollte in der Größenordnung der Länge des halben Abstandes gegenüberliegender, durch die Trennwand getrennter Magnete M1 bis M4 liegen. Größenordnungsmäßig kommt ein prozentualer Unterschied der beiden Längen von bis zu 50% in Frage.

Die Vorrichtung zur magnetischen Lagerentlastung besteht im wesentlichen aus den Platten 21, 23, die stationär sein müssen, und den Magneten M1 bis M4 mit den zugeordneten Luftspalten L. Anhand der Kraftpfeile für die Axialkraft $F_{ax}$ ist zu erkennen, daß auf die Wellen 11, 13 bei korrekter Einstellung der Luftspalte L keine resultierende Axialkraft wirkt. Die Kraft $F_{ax}$, mit der sich die Magnete M1, M3 bzw. M2, M4 jeweils anziehen, wird nämlich kompensiert durch eine entgegengerichtete zweite Kraft $F_{ax}$ von gleicher Größe, mit welcher die Magnete M1 bis M4 jeweils zu den Platten 21, 23 hingezogen werden. Dieses geschieht aufgrund ihres eigenen Magnetfeldes, so daß keine weiteren Bauteile für die Lagerentlastung nötig sind. Über die Platten 21, 23 schließt sich der magnetische Fluß.

In Figur 2 ist der Versatz der Magnete untereinander erkenntlich. Die diagonal verlaufende Kraft F, mit der sich die Magnete M1, M3 anziehen, läßt sich aufteilen in eine Kraftkomponente $F_{ax}$, die in Axialrichtung der Magnete M1 bis M4 verläuft, und in eine Kraft $F_{tan}$, die in Tangentialrichtung der gezeigten Stirnmagnetdrehkupplung verläuft. Wie bereits vorher beschrieben, wird die Kraft $F_{ax}$ bei jedem der Magnete M1 bis M4 kompensiert, so daß lediglich noch die Kraft $F_{tan}$ übrigbleibt. Diese dient zur Übertragung des Drehmoment. Je nach Größe der diagonalen Kraft F, die sich aus dem gewünschten Betriebszustand der Stirndrehkupplung ergibt, wird der Abstand zwischen der Platte 21, 23 und den Magneten M1, M2 bzw. M3, M4 eingestellt. Der gewünschte Betriebszustand wird dabei bauseitig aufgrund der gegebenen Werte für die Übertragungsparameter, wie z.B. den Pumpwiderstand, die gewünschte Motordrehzahl festgelegt. Ist dieser Betriebszustand und der zugehörige Luftspalt L einmal eingestellt, bedarf es keiner weiteren Änderungen mehr.

Vorteil der gezeigten Stirndrehkupplung für eine Dosiervorrichtung ist es, daß die Lagerentlastung ohne den Einbau von weiteren Bauteilen – außer der Platte 21, 23 – bereitgestellt wird. Dieses ist besonders bei den engen Platzverhältnissen in implantierbaren Medikamentendosiergeräten von Vorteil. Dieser Effekt der Platzeinsparung kann noch dadurch verstärkt werden, daß die Platte 21 als Teil des Antriebs- oder Motorgehäuses ausgelegt wird. Sie kann nämlich direkt eine Stirnseite des Motorgehäuses bilden, wobei dann die Aussparung 27 die Wellendurchführung des Motors ist. Analog ist es möglich, die zweite Platte 23 als Teil des Antriebsgehäuses oder des Pumpgetriebes auszubilden. In beiden Fällen ist es wichtig, daß die Platte 21, 23 stationär gegenüber den Magneten M1 bis M4 befestigt ist.

Die Anwendung der magnetischen Lagerentlastung ist nicht auf Stirnmagnetdrehkupplungen beschränkt. Insbesondere ist die Lagerentlastung auch bei einer translatorisch wirkenden Magnet-Verschiebungskupplung einsetzbar. Die Platte 21 ist dann als langgestrecktes Teil auszuführen und in Längsrichtung der Verschieberichtung anzuordnen.

## Patentansprüche

1. Stirnmagnetkupplung (1), versehen mit mindestens einem Magneten (M1–M4) zur Übertragung von Drehmomenten oder Kräften von einem ersten (15) in einen zweiten (17) Raum, wobei die beiden Räume (15, 17) voneinander durch eine Trennwand (19) getrennt sind, dadurch gekennzeichnet, daß die Stirnmagnetkupplung (1) zumindest in einem der Räume (15, 17) mit einer Einrichtung zur magnetischen Lagerentlastung durch as Magnetfeld des Magneten (M1–M4) selbst versehen ist, die aus einer über der freien Stirnseite des Magneten (M1–M4) und von diesem durch einen Luftspalt (L) getrennten, gegenüber dam magneten stationär angeordneten Platte (21, 23) aus einem magnetisierbaren Material besteht.

2. Stirnmagnetkupplung nach Anspruch 1, dadurch gekennzeichnet, daß die Platte (21, 23) stationär an einem Gehäuseteil oder einem am Gehäuse befestigten Geräteteil angebracht ist.

3. Stirnmagnetkupplung nach Anspruch 2, dadurch gekennzeichnet, daß das Geräteteil ein mit der Magnetkupplung (1) verbundener Antrieb ist, und daß eine Stirnseite des Antriebgehäuses als die Platte (21, 23) ausgebildet ist.

4. Stirnmagnetkupplung nach Anspruch 2 oder 3, dadurch gekennzeichnet, daß das Geräteteil ein mit der Magnetkupplung (1) verbundener Abtrieb ist, und daß eine Stirnseite des Abtriebsgehäuses als die Platte (21, 23) ausgebildet ist.

5. Stirnmagnetkupplung nach einem der Ansprüche 1 bis 4, dadurch gekennzeichnet, daß die Länge des Luftspaltes (L) in der Größenordnung der Länge des halben Abstandes von zwei gegenüberliegenden, durch die Trennwand (19) getrennten Magneten (M1–M4) liegt.

6. Stirnmagnetkupplung nach Anspruch 5, dadurch gekennzeichnet, daß die Länge des Luftspaltes (L) bis zu 50% vom halben Abstand von zwei gegenüberliegenden, durch die Trennwand (19) getrennten Magneten (M1–M4) abweicht.

7. Stirnmagnetkupplung nach Anspruch 6, dadurch gekennzeichnet, daß die Platte (21, 23) aus Nickel, Weicheisen oder einem anderen weichmagnetischen Werkstoff besteht.

8. Stirnmagnetkupplung nach einem der Ansprüche 1 bis 7, dadurch gekennzeichnet, daß die Platte (21, 23) mit einer Aussparung (27, 29) zur Durchführung der Antriebs- bzw. Abtriebswelle versehen ist.

9. Stirnmagnetkupplung nach einem der Ansprüche 1 bis 8, dadurch gekennzeichnet, daß die Magnetkupplung (1) eine Magnetträgerplatte (7, 9) aus unmagnetisiertem Material umfaßt, daß mindestens der eine Magnet (M1) sowie eine Welle (11) an diesem Magnetträger (7) befestigt sind, wobei die Welle (11) durch eine Aussparung (27) in einer stationär angeordneten Platte (21) hindurchgeführt ist, die als magnetischer Rückschluß für den Magneten (M1) vorgesehen ist, und daß zwischen der Magnetträgerplatte (7) und der Platte (21) ein Luftspalt (L) von vorgegebener Breite (b) vorgesehen ist.

10. Stirnmagnetkupplung nach Anspruch 9, dadurch gekennzeichnet, daß die Magnetträgerplatte (7, 9) eine runde Scheibe ist, an der diagonal zueinander mindestens zwei Magnete (M1, M2 bzw. M3, M4) befestigt sind, wobei nebeneinanderliegende Magnete gegensinnig magnetisiert sind.

11. Verwendung einer Stirnmagnetkupplung nach einem der Ansprüche 1 bis 10, in einem Medikamentendosiergerät.

## Claims

1. End face magnetic coupling (1), provided with at least one magnet (M1–M4) for the transmission of turning moments or forces from a first compartment into a second compartment, the two compartments (15, 17) being separated from each other by means of a partition wall (19), characterised in that the end face magnetic coupling (1) is provided, at least in one of the compartments (15, 17), with a device for magnetic bearing flotation through the magnetic field of the magnet (M1–M4) itself, which device consists of a plate (21, 23), which is separated above the free end face of the magnet (M1–M4) and separated from the magnet by means of an air gap (L), is arranged such that it is stationary in relation to the magnet and is made of a magnetizable material.

2. End face magnetic coupling according to claim 1, characterised in that the plate (21, 23) is provided in a stationary manner on a part of the housing or on a part of the apparatus secured to the housing.

3. End face magnetic coupling according to claim 2, characterised in that the part of the apparatus is a drive element connected with the magnetic coupling (1) and in that an end face of the drive element housing forms the plate (21, 23).

4. End face magnetic coupling according to claim 2 or 3, characterised in that the part of the apparatus is a driven element connected with the magnetic coupling (1) and in that an end face of the driven element housing forms the plate (21, 23).

5. End face magnetic coupling according to one of the claims 1 to 4, characterised in that the length of the air gap (L) is of the order of magnitude of half of the length of the spacing of two facing magnets (M1–M4) separated by the partition wall (19).

6. End face magnetic coupling according to claim 5, characerised in that the length of the air gap (L) differs, by less than 50%, from half the spacing of two facing magnets (M1–M4) separated by the partition wall (19).

7. End face magnetic coupling according to claim 6, characterised in that the plate (21, 23) is made of nickel, soft iron or another soft magnetic material.

8. End face magnetic coupling according to one of the claims 1 to 7, characterised in that the plate (21, 23) is provided with a recess (27, 29) for the through–guidance of the drive or driven shaft.

9. End face magnetic coupling according to one of the claims 1 to 8, characterised in that the magnetic coupling (1) comprises a magnet carrier plate (7, 9) of non-magnetized material, in that at least one magnet (M1) and also one shaft (11) are secured to this magnet carrier (7), the shaft (11) being guided through a recess (27) in a plate (21) which is arranged in a stationary manner and is provided as a magnetic return path for the magnet (M1), and in that an air gap (L) of given width (b) is provided between the magnet carrier plate (7) and the plate (21).

10. End face magnetic coupling according to claim 9, characterised in that the magnet carrier plate (7, 9) is a round disc, to which there are secured, diametrically opposite one another, at least two magnets (M1, M2 or M3, M4), adjacent magnets being magnetized in oppositions

11. Use of an end face magnetic coupling according to one of the claims 1 to 10 in a medicine-dosing apparatus.

## Revendications

1. Accouplement magnétique frontal (1), comprenant au moins un aimant (M1–M4) pour transmettre des couples ou des forces d'un premier espace (15) à un second espace (17), les deux espaces (15, 17) étant séparés l'un de l'autre par une cloison (19), caractérisé en ce qu'il est muni, dans au moins l'un des deux espaces (15, 17) d'un dispositif pour le soulagement magnétique du palier par le champ magnétique de l'aimant (M1–M4) lui-même, dispositif qui est constitué d'une plaque (21, 23) en matériau magnétisable, laquelle est disposée en regard du côté frontal libre de l'aimant (M1–M4) dont elle est séparée par un entrefer (L).

2. Accouplement selon la revendication 1, caractérisé en ce que la plaque (21, 23) est disposée stationnaire sur une partie de carter ou sur une partie d'appareil fixée au carter.

3. Accouplement selon la revendication 2, caractérisé en ce que la partie d'appareil est un dispositif d'entraînement relié à l'accouplement magnétique (1) et qu'un côté frontal du carter menant est réalisé pour constituer la plaque (21, 23).

4. Accouplement selon la revendication 2 ou 3, caractérisé en ce que la partie d'appareil est un dispositif mené relié à l'accouplement magnétique (1) et qu'un côté frontal du carter mené est réalisé pour constituer la plaque (21, 23).

5. Accouplement selon une des revendications 1 à 4, caractérisé en ce que la longueur de l'entrefer (L) est de l'order de grandeur de la longueur de la moitié de l'espacement de deux aimants (M1–M4) opposés, séparés par la cloison (19).

6. Accouplement selon la revendication 5, caractérisé en ce que la longueur de l'entrefer (L) s'écarte jusqu'à 50% du demi espacement de deux aimants (M1–M4) opposés, séparés par la cloison (19).

7. Accouplement selon la revendication 6, caractérisé en ce que la plaque (21, 23) est en nickel, fer doux ou un autre matériau magnétique doux.

8. Accouplement selon une des revendications 1 à 7, caractérisé en ce que la plaque (21, 23) est pourvue d'un évidement (27, 29) pour la traversée de l'arbre menant ou de l'arbre mené.

9. Accouplement selon une des revendications 1 à 8, caractérisé en ce que l'accouplement magnétique (1) comprend une plaque porte-aimants (7, 9) en matériau non magnétisé, qu'au moins ledit aimant (M1) et un arbre (11) sont fixés à ce porte-aimant (7), avec passage de l'arbre (11) à travers un évidement (27)) ménagé dans une plaque (21) disposée stationnaire et qui sert d'élément de fermeture du circuit magnétique pour l'aimant (M1), et qu'un entrefer (L) de largeur (b) préfixée est prévu entre la plaque porte-aimant (7) et la plaque (21).

10. Accouplement selon la revendication 9, caractérisé en ce que la plaque porte-aimant (7, 9) est un disque rond auquel sont fixés au moins deux aimants (M1, M2 ou M3, M4) disposés diagonalement l'un par rapport à l'autre, des aimants placés l'un à côté de l'autre étant aimantés en sens contraire.

11. Utilisation d'un accouplement magnétique frontal selon une des revendications 1 à 10 dans un appareil doseur de médicaments.

FIG 1

FIG 2